(19) **Europäisches Patentamt / European Patent Office / Office européen des brevets**

(11) **EP 4 552 646 A1**

(12) **EUROPEAN PATENT APPLICATION**
published in accordance with Art. 153(4) EPC

(43) Date of publication:
**14.05.2025 Bulletin 2025/20**

(21) Application number: 23865904.9

(22) Date of filing: **15.09.2023**

(51) International Patent Classification (IPC):
**A61K 39/00** *(2006.01)* **A61K 35/15** *(2025.01)*
**A61K 35/17** *(2025.01)*

(52) Cooperative Patent Classification (CPC):
**A61K 35/15; A61K 35/17; A61K 39/00; A61P 35/00**

(86) International application number:
**PCT/KR2023/013932**

(87) International publication number:
**WO 2024/058609 (21.03.2024 Gazette 2024/12)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **15.09.2022 KR 20220116552**

(71) Applicant: **Cellid Co., Ltd.
Seoul 08826 (KR)**

(72) Inventors:
• **KANG, Chang-Yuil**
Seoul 08826 (KR)
• **SONG, Boyeong**
Seoul 08826 (KR)
• **JEON, Insu**
Seoul 08826 (KR)

(74) Representative: **Office Freylinger**
P.O. Box 48
**8001 Strassen (LU)**

(54) **VACCINE COMPRISING PERIPHERAL BLOOD MONONUCLEAR CELLS LOADED WITH NATURAL KILLER T CELL LIGAND AND ANTIGEN**

(57)    The present invention relates to an immunoprophylactic and therapeutic vaccine comprising peripheral blood mononuclear cells loaded with a natural killer T cell ligand and an antigen, and specifically, to an immunotherapeutic vaccine comprising peripheral blood mononuclear cells loaded with alpha-galactosylceramide, a natural killer T cell ligand and a type of glycolipid. A composition of the present invention is easy to obtain because there is no need to separate specific cells from peripheral blood mononuclear cells. In addition, immunization of peripheral blood mononuclear cells loaded with a natural killer T cell ligand and an antigen not only induces significant levels of activation of natural killer cells and natural killer T cells and cytotoxic T lymphocyte responses, but also has a synergistic effect in the treatment of malignant tumors and thus can be helpfully used as an anticancer immunotherapeutic agent.

EP 4 552 646 A1

**Description**

**BACKGROUND OF THE INVENTION**

**1. Field of the Invention**

Cross Reference to Related Application

This application claims the benefit of Korean Patent No. 10-2022-0116552, filed September 15, 2022, which is herein incorporated by reference in its entirety.

Field of the Invention

The present invention relates to a vaccine comprising peripheral blood mononuclear cells loaded with natural killer T cell ligands and antigens, for example, cancer antigens.

**2. Description of the Related Art**

Recent advances in medicine have increased the survival rate of cancer patients, but the incidence of cancer is also increasing due to changing environmental factors and an increase in life expectancy. To date, much research is being conducted to treat cancer, and new drugs and treatment methods for cancer are being developed, which is greatly improving the treatment effectiveness for cancer patients. However, in the case of malignant tumors, the therapeutic effects are limited despite the development of microsurgical therapy and radiotherapy, and the development of new therapeutic agents for chemotherapy, and there are limitations such as side effects due to non-specific anticancer effects and recurrence of cancer. Immunotherapy is a treatment method that is currently being actively used or developed to complement this. Immunotherapy has the characteristics to complement conventional cancer therapies by inducing tumor-specific toxicity, reducing side effects of systemic toxicity, and establishing an active memory response to cancer and cancer antigens.

Immunotherapy, especially an immune cell vaccine using antigen-presenting cells, can effectively activate CD8+ T cells and CD4+ T cells, and thus demonstrating excellent anticancer effects.

Currently, the most commonly used immune cells in antigen-presenting cell vaccines are dendritic cells, which phagocytize antigens and deliver them to effector cells such as T cells along with strong co-stimulatory signals, thereby efficiently activating effector cells and inducing strong immune responses. In actual clinical cell therapy using dendritic cells, first, dendritic cells are isolated from the patient's bone marrow or peripheral blood, or their precursors, monocytes, are isolated, and then mass-proliferated and differentiated into dendritic cells, after which antigens are added and activated, and then injected back into the patient. Once injected into the body, the dendritic cells transmit specific antigen information to T cells and activate them, effectively inducing an antigen-specific immune response. Despite these advantages, dendritic cells have not been widely used in antigen-presenting cell vaccines because very few dendritic cells can be obtained directly from blood and lymphoid tissue, they are difficult to isolate, and when differentiated from monocytes, they must be cultured in vitro for several days.

In a previous study, the present inventors confirmed the effect of B cells loaded with $\alpha$-GC on inducing cytotoxic T lymphocyte responses (Korean Patent No. 10-0809873). Because B cells exist in large amounts in lymphoid tissues and blood and can be easily proliferated in vitro, they are an effective substitute for dendritic cells for cell vaccines, and have the advantage of migrating to lymphoid organs after intravenous administration. Despite these advantages, B cells have not been widely used in antigen-presenting cell vaccines due to their weak immunogenicity.

On the other hand, it has been well known through recent studies that invariant natural killer T cells (iNKT cells) play a crucial role in controlling various immune responses and immunopathological phenomena. Ligand-activated invariant natural killer T cells can induce activation of T cells, B cells, and natural killer cells as well as dendritic cells.

Cancer cells loaded with $\alpha$-GC, a ligand of natural killer T cells, can induce cancer antigen-specific T cell activity. It is known that the expressions of IFN-$\gamma$ and granzyme B are strongly induced in mice administered with T cells loaded with $\alpha$-GC and antigen peptides, and that anticancer therapeutic effects are exhibited in mice with lung metastatic cancer through the activation of antigen-specific cytotoxic T lymphocyte response (Yeonseok Chung et al., OncoImmunology, 1:2, 141-151, 2012).

The present inventors have created an effective cell therapy vaccine by confirming that an active immune response against a cancer antigen presented by the major histocompatibility complex of B cells can be generated by loading B cells with natural killer T cell ligands to change the autoimmune tolerance of these cells to immunogenicity, and exhibit anticancer effects.

In addition, the present inventors have produced an effective cell therapy vaccine by confirming in previous studies that when α-GC-loaded monocytes are loaded with antigen peptides or transduced with antigen-expressing adenovirus and administered into the body, an antigen-specific immune response is induced and significant anticancer effects are exhibited (Korean Patent No. 10-1055666).

However, the cell therapy agents for clinical trial developed from the above prior studies are produced using a manufacturing process that removes T cells from a patient's peripheral blood, leaving B cells and monocytes. This method has the disadvantage that it is difficult to separate T cells, B cells, and monocytes from peripheral blood, respectively, and the manufacturing process is complicated.

To solve these problems, the present inventors have developed an anticancer immunotherapeutic and preventive vaccine by loading natural killer T cell ligands and antigens into peripheral blood mononuclear cells obtained without isolating each immune cells based on the studies on the various immune cells mentioned above.

Peripheral blood mononuclear cells are a general term for the group of mononuclear mesenchymal cells present in the bloodstream, and are mainly composed of monocytes and lymphocytes. In general, peripheral blood mononuclear cells refer to the white blood cell fraction obtained by specific gravity centrifugating blood and excluding polymorphonuclear leukocytes. Lymphocytes include T cells, B cells, and natural killer cells (NK cells). Specifically, the cell therapy agent developed by the present inventors uses peripheral blood mononuclear cells, which are cells with round nuclei composed of lymphocytes (T cells, B cells, and natural killer cells) and monocytes, as a raw material. The proportion of each type of immune cell in peripheral blood mononuclear cells varies greatly from person to person, but is generally composed of 45-70% T cells, 5-15% B cells, 5-10% natural killer cells, and 5-10% monocytes.

The present inventors have made efforts to solve the problems of dendritic cell vaccines differentiated from dendritic cells and monocytes and the problems of prior inventions using B cells and monocytes, and as a result, have confirmed that a vaccine using peripheral blood mononuclear cells has a synergistic effect superior to the immune effects of B cells, monocytes, and T cells, respectively, and have completed the development of the vaccine comprising peripheral blood mononuclear cells of the present invention.

In addition, the cell therapy agent of the present invention has various advantages over previous cell therapy agents, including increasing the amount of peripheral blood mononuclear cells, a raw material, through a process that does not isolate each type of immune cells. If the process of staining and removing T cells in the manufacturing process of existing cell therapy agents is eliminated, the peripheral blood mononuclear cells used as a raw material will increase and the final production volume will increase, leading to time, labor, and cost savings and increased manufacturing volume.

## SUMMARY OF THE INVENTION

It is an object of the present invention to provide an immunotherapeutic or preventive vaccine comprising peripheral blood mononuclear cells loaded with natural killer T cell ligands and antigens.

It is another object of the present invention to provide a method for producing an immunotherapeutic or preventive vaccine comprising peripheral blood mononuclear cells loaded with natural killer T cell ligands and antigens.

To achieve the above objects, the present invention provides an immunotherapeutic or preventive vaccine comprising peripheral blood mononuclear cells loaded with natural killer T cell ligands and antigens.

In another aspect of the present invention, the present invention provides a method for producing an immunotherapeutic or preventive vaccine comprising peripheral blood mononuclear cells loaded with natural killer T cell ligands and antigens.

The present invention also provides a pharmaceutical composition for preventing or treating cancer, which comprises peripheral blood mononuclear cells loaded with natural killer T cell ligands and antigens as an active ingredient.

The present invention also provides a use of peripheral blood mononuclear cells loaded with natural killer T cell ligands and cancer antigens for preventing or treating cancer.

In addition, the present invention provides a method for preventing or treating cancer by administering an effective amount of peripheral blood mononuclear cells loaded with natural killer T cell ligands and antigens to a subject.

## ADVANTAGEOUS EFFECT

The present invention relates to a vaccine for immunoprophylaxis and immunotherapy, comprising peripheral blood mononuclear cells loaded with natural killer T cell ligands and antigens, particularly to an immunotherapeutic vaccine comprising peripheral blood mononuclear cells loaded with alpha-galactosylceramide (α-GC), a natural killer T cell ligand and a type of glycolipid. The composition of the present invention is easy to obtain because there is no need to isolate specific cells from peripheral blood mononuclear cells, and immunization of peripheral blood mononuclear cells loaded with natural killer T cell ligands and antigens not only induces significant levels of natural killer cell and natural killer T cell activation and cytotoxic T lymphocyte responses but also exhibits synergistic therapeutic effects on malignant tumors so that it can be effectively used as an anticancer immunotherapy agent.

## BRIEF DESCRIPTION OF THE DRAWINGS

Figure 1 is a diagram showing the manufacturing process of a peripheral blood mononuclear cell vaccine according to an embodiment of the present invention.

Figure 2A is a set of graph showing the activity of natural killer T cells (NKT) and natural killer cells (NK) in the spleen of a mouse following administration of a peripheral blood mononuclear cell vaccine transduced with adenoviruses loaded with $\alpha$-GC and/or expressing antigens.

Figure 2B is a graph showing the IFN-$\gamma$ production by natural killer T cells (NKT) of Figure 2A.

Figure 2C is a graph showing the IFN-$\gamma$ production by natural killer cells (NK) of Figure 2A.

Figure 3A is a diagram showing the experimental method for comparing the activation of cytotoxic T cells in mice by administration of a peripheral blood mononuclear cell vaccine transduced with adenoviruses loaded with $\alpha$-GCs and/or expressing antigens (target peptide: MAGE-A3$_{282-290}$).

Figure 3B is a set of histograms showing the results of measuring the activity of antigen-specific cytotoxic T cells induced by a peripheral blood mononuclear cell vaccine transduced with adenoviruses loaded with $\alpha$-GC and/or expressing antigens.

Figure 3C is a graph showing the activity of antigen-specific cytotoxic T cells induced by a peripheral blood mononuclear cell vaccine transduced with adenoviruses loaded with $\alpha$-GC and/or expressing antigens.

Figure 4A is a diagram showing the experimental method for confirming the therapeutic effect of a peripheral blood mononuclear cell vaccine transduced with adenoviruses loaded with $\alpha$-GC and/or expressing antigens on mouse solid cancer.

Figure 4B is a graph showing the anticancer effect (change in tumor size) of a peripheral blood mononuclear cell vaccine transduced with adenoviruses loaded with $\alpha$-GC and/or expressing antigens.

Figure 4C is a graph showing the anticancer effect (change in survival rate) of a peripheral blood mononuclear cell vaccine transduced with adenoviruses loaded with $\alpha$-GC and/or expressing antigens.

Figure 5A is a diagram showing the experimental method for confirming the therapeutic effect of a peripheral blood mononuclear cell vaccine transduced with adenoviruses loaded with $\alpha$-GC and/or expressing antigens on lung metastatic cancer.

Figure 5B is a set of photographs showing the lungs of a lung metastasis cancer model mouse administrated with a peripheral blood mononuclear cell vaccine transduced with adenoviruses loaded with $\alpha$-GC and/or expressing antigens.

Figure 5C is a graph showing the therapeutic effect (number of metastatic nodules) of a peripheral blood mononuclear cell vaccine transduced with adenoviruses loaded with $\alpha$-GC and/or expressing antigens on lung metastatic cancer.

Figure 6A is a diagram showing the experimental method for confirming the therapeutic effect of a peripheral blood mononuclear cell vaccine transduced with adenoviruses loaded with $\alpha$-GC and/or expressing antigens on metastatic cancer.

Figure 6B is a graph showing the anticancer effect (change in survival rate) of a peripheral blood mononuclear cell vaccine transduced with adenoviruses loaded with $\alpha$-GC and/or expressing antigens.

Figure 7A is a diagram showing the experimental method for confirming the activation of antigen-specific cytotoxic T cells by a peripheral blood mononuclear cell vaccine co-loaded with $\alpha$-GC and HER2$_{63-71}$ peptide (target peptide: HER2$_{63-71}$).

Figure 7B is a set of histograms showing the activity of antigen-specific cytotoxic T cells induced by a peripheral blood mononuclear cell vaccine co-loaded with $\alpha$-GC and HER2$_{63-71}$ peptide.

Figure 7C is a graph showing the activity of antigen-specific cytotoxic T cells induced by peripheral blood mononuclear cells co-loaded with $\alpha$-GC and HER2$_{63-71}$ peptide.

Figure 8A is a diagram showing the experimental method for confirming the therapeutic effect of a peripheral blood mononuclear cell vaccine loaded with $\alpha$-GC and/or a cancer antigen peptide on mouse solid cancer (target peptide: HER2$_{63-71}$).

Figure 8B is a graph showing the anticancer effect (change in tumor size) of a peripheral blood mononuclear cell vaccine loaded with $\alpha$-GC and/or a cancer antigen peptide.

Figure 9A is a diagram showing the experimental method for comparing the activation of antigen-specific cytotoxic T cells by a peripheral blood mononuclear cell vaccine transduced with adenoviruses loaded with $\alpha$-GC and/or expressing antigens and a B cell/monocyte vaccine (target peptide: GP100$_{25-33}$).

Figure 9B is a set of histograms showing the activity of antigen-specific cytotoxic T cells induced by a peripheral blood mononuclear cell vaccine transduced with adenoviruses loaded with $\alpha$-GC and/or expressing antigens and a B cell/monocyte vaccine.

Figure 9C is a graph showing the activity of antigen-specific cytotoxic T cells induced by a peripheral blood mononuclear cell vaccine transduced with adenoviruses loaded with $\alpha$-GC and/or expressing antigens and a B cell/monocyte vaccine.

Figure 10A is a diagram showing the experimental method for comparing the activation of antigen-specific cytotoxic T cells by a peripheral blood mononuclear cell vaccine transduced with adenoviruses loaded with $\alpha$-GC and/or expressing antigens, a B cell/monocyte vaccine, and a T cell vaccine (target peptide: $GP100_{25-33}$).

Figure 10B is a set of histograms showing the activity of antigen-specific cytotoxic T cells induced by a peripheral blood mononuclear cell vaccine transduced with adenoviruses loaded with $\alpha$-GC and/or expressing antigens, a B cell/monocyte vaccine, and a T cell vaccine.

Figure 10C is a graph showing the activity of antigen-specific cytotoxic T cells induced by a peripheral blood mononuclear cell vaccine transduced with adenoviruses loaded with $\alpha$-GC and/or expressing antigens, a B cell/monocyte vaccine, and a T cell vaccine.

Figure 11A is a diagram showing the experimental method for comparing the therapeutic effects of a peripheral blood mononuclear cell vaccine transduced with adenoviruses loaded with $\alpha$-GC and/or expressing antigens and a B cell/monocyte vaccine on solid cancer.

Figure 11B is a set of graphs showing the anticancer effect (change in tumor size) of a peripheral blood mononuclear cell vaccine transduced with adenoviruses loaded with $\alpha$-GC and/or expressing antigens and a B cell/monocyte vaccine.

## DESCRIPTION OF THE PREFERRED EMBODIMENTS

Hereinafter, the present invention is described in detail.

The present invention provides an immunotherapeutic or preventive vaccine for cancer comprising peripheral blood mononuclear cells loaded with natural killer T cell ligands and cancer antigens.

The peripheral blood mononuclear cells refer to cells with round nuclei comprising lymphocytes (T cells, B cells, and natural killer cells) and monocytes. The proportion of each type of immune cell in peripheral blood mononuclear cells varies greatly from person to person, but is generally composed of 45-70% T cells, 5-15% B cells, 5-10% natural killer cells, and 5-10% monocytes.

Herein, the present inventors have developed an anticancer therapeutic cell vaccine that can induce a significantly enhanced anticancer immune response by adding the function of antigen-presenting cells by altering the immunogenicity of immune cells through loading of $\alpha$-GC, a natural killer T cell ligand, and antigen delivery to immune cells.

Neoepitopes, the peptide sequences that are created by highly tumor-specific mutations that exist only in cancer cells, not normal cells, and can induce cancer cell-specific immune responses, are known as ideal targets for personalized anticancer immunotherapy for cancer patients. Anticancer immunotherapy using an immune cell vaccine that delivers neoepitopes discovered for personalized treatment for cancer patients is expected to induce the generation of more cancer cell-specific T cells than conventional immunotherapy, thereby minimizing the side effects of anticancer treatment by preventing damage to normal cells, while simultaneously inducing a strong anticancer treatment effect.

Alpha-galactosyl ceramide ($\alpha$-GC) is an immune enhancer that induces various anticancer immune responses by stimulating NKT cells. In particular, it induces T cell immune responses by allowing B cells and monocytes to stimulate T cells with the same efficiency as dendritic cells.

It is well known that dendritic cells (DCs) loaded with alpha-galactosylceramide ($\alpha$-GC) activate invariant natural killer T (NKT) cells (van der Vliet HJ, et al., J Immunol Methods., 1;247(1-2):61-72, 2001). The present inventors confirmed the effect of inducing cytotoxic T lymphocyte responses in B cells, monocytes, and immature myeloid cells loaded with $\alpha$-GC (Korean Patent Publication No. 10-2007-0105662 and Korean Patent Publication No. 10-2009-0051598). In addition, it was confirmed that $\alpha$-GC-loaded natural killer cells activated natural killer T (NKT) cells and induced cytotoxic T lymphocyte responses (Korean Patent No. 10-2022-0072485).

Accordingly, the present inventors produced an anticancer vaccine with enhanced immune function by adding the function of antigen-presenting cells by changing the immunogenicity of immune cells through $\alpha$-GC loading and antigen delivery to immune cells.

Meanwhile, antigen delivery using viral vectors is suitable for large-scale production of cell therapy agents targeting cancers expressing specific cancer antigens by delivering cancer antigens to cells with high efficiency. When a virus capable of expressing the above antigen is used, the entire antigen can be introduced, so it is applicable to all people without being limited to a specific haplotype of the major histocompatibility complex, and has the advantage of being able to induce not only a cellular immune response but also a humoral immune response. On the other hand, viral vectors are limited in their application to personalized cancer therapy.

The method of antigen delivery through peptide loading, when used clinically, is limited to the haplotype of an individual's major histocompatibility complex (MHC), so it cannot be used universally, and has the disadvantage of presenting only a single epitope. However, neoepitopes, the peptide sequences that are created by highly tumor-specific mutations that exist only in cancer cells, not normal cells, and can induce cancer cell-specific immune responses, are known as ideal targets for personalized anticancer immunotherapy for cancer patients. Anticancer immunotherapy using an immune cell vaccine that delivers neoepitopes discovered for personalized treatment for cancer patients is expected to induce the generation of more cancer cell-specific T cells than conventional immunotherapy.

The ligands of the above natural killer T cells include alpha-galactosylceramide ($\alpha$-GC), alpha-glucuronosylceramide, phosphatidylinositol tetramannoside, isoglobotrihexosylceramide, ganglioside GD3, phosphatidylcholine, phosphatidylethanolamine, phosphatidylinositol, sulfitide, beta-galactosylceramide, lipophosphoglycan, glycoinositol phospholipids, beta-anomeric galactosylceramide and alpha-anomeric galactosylceramide, which are analogues of alpha-galactosylceramide, bacterial lipid antigens, and variants of alpha-galactosylceramide.

The above antigen may be any antigen that can be used as a vaccine and induce an immune response, and includes antigens derived from pathogens including pathogenic bacteria, viruses, and parasites, or cancer antigens, and can be the full length or a fragment of the above antigen.

The antigens derived from the pathogenic bacteria include Bordetella pertussis antigens (pertussis toxin, filamentous haemagglutinin, and pertactin), tetanus toxoid, diphtheria toxoid, Helicobacterpylori antigens (capsular polysaccharides of serogroups A, B, C, Y, and W-135), pneumococcal antigen (Streptococcus pnemoniae type 3 capsular polysaccharide), tuberculosis antigen, cholera antigen (cholera toxin B subunit), staphylococcal antigen (staphylococcal enterotoxin B), shigella antigen (shigella polysaccharides), Borrelia sp. antigen, Candida albicans antigen, and Plasmodium antigen.

The antigens derived from the viruses include influenza virus antigens (haemagglutinin and neuraminidase), human papilloma virus (HPV) antigen (glycoprotein), vesicular stomatitis virus antigen (vesicular stomatitis virus glycoprotein), cytomegalovirus (CMV) antigen, hepatitis virus antigens (hepatitis A(HAV), B(HBV), C(HCV), D(HDV) and G(HGV) antigens) (core antigen and surface antigen), respiratory synctytial virus (RSV) antigen, herpes simplex virus antigen, human immunodeficiency virus (HIV) antigens (GP-120, GP-160, p18, Tat, Gag, Pol, Env) and combinations thereof.

The cancer antigens include gp100, melanoma antigen gene (MAGE), human papilloma virus (HPV) E6/E7, tyrosinase, tyrosinase-related protein-1 (TRP-1), tyrosinase-related protein-2 (TRP-2), murinoglobulin 1 (MUC-1), carcinoembryonic antigen (CEA), p53, $\alpha$-fetoprotein, breast cancer protein expressed by Her-2/neu, proteinase 3, WT-1, PAP, PSA, PSMA, G250, BAGE, GAGE, NY-ESO- 1, MART-1, MCIR, Ig Idiotype, CDK4, caspase-8, $\beta$-catenin, CIA, BCR/ABL, EBV LMP2a, HCV, HHV-8, 5T4, tumor-specific mutation-derived neoantigens and combinations thereof.

The vaccine may be used for the treatment or prevention of cancer.

The above cancer may include all types of cancer. The cancer includes liver cancer, thyroid cancer, testicular cancer, bone cancer, glioblastoma, oral cancer, ovarian cancer, brain tumor, multiple myeloma, gallbladder cancer, biliary tract cancer, colon cancer, head and neck cancer, lymphoma, bladder cancer, leukemia, esophageal cancer, kidney cancer, stomach cancer, breast cancer, cervical cancer, prostate cancer, rectal cancer, spinal cord tumor, pancreatic cancer, salivary gland cancer, lung cancer, skin cancer, laryngeal cancer, melanoma, acute myeloid leukemia, neuroblastoma, retinoblastoma, colorectal cancer, etc.

The cancer may be solid cancer or metastatic cancer.

In addition, the antigen may be directly loaded into peripheral blood mononuclear cells in the form of peptides, lipopolysaccharides, polysaccharides, glycoproteins, or polynucleotides including DNA and RNA, and can be transfected into peripheral blood mononuclear cells by recombinant viruses and expressed and loaded. Compared to the cell vaccine loaded with peptides, the cell vaccine introduced with the entire antigen via viruses are applicable to all people, not limited to the haplotype of the major histocompatibility complex, and can induce immune responses specific to multiple epitopes, and in particular, has the advantage of being able to induce humoral and cellular immune responses simultaneously.

The vaccine of the present invention can additionally include, in addition to the natural killer T cell ligands and peripheral blood mononuclear cells, one or more effective ingredients having the same or similar effect with them.

The vaccine can also include, in addition to the above-mentioned effective ingredients, one or more pharmaceutically acceptable carriers for the administration. The pharmaceutically acceptable carrier can be selected or be prepared by mixing more than one ingredients selected from a group consisting of saline, Ringer's solution, buffered saline, dextrose solution, maltodextrose solution, glycerol, and ethanol. Other general additives such as anti-oxidative agent, buffer solution, bacteriostatic agent, etc., can be added. In order to prepare injectable solutions such as aqueous solution, suspension and emulsion, diluents, dispersing agents, surfactants, binders and lubricants can be additionally added. The vaccine of the present invention can further be prepared in suitable forms for each disease or according to ingredients by following a method represented in Remington's Pharmaceutical Science (the newest edition), Mack Publishing Company, Easton PA.

The vaccine of the present invention can be administered by parenterally and the parenteral administration includes subcutaneous injection, intravenous injection, intramuscular injection and intrathoracic injection. To prepare the vaccine as a formulation for parenteral administration, the peripheral blood mononuclear cells loaded with ligands of natural killer T cells, the peripheral blood mononuclear cells loaded with ligands of natural killer T cells and peptides, or the peripheral blood mononuclear cells infected with viruses expressing cancer antigens of the present invention is mixed with a stabilizer or a buffering agent to produce a solution or suspension, which is then formulated as ampoules or vials.

The vaccine of the present invention can be formulated in a variety of forms according to administration pathways. For example, the vaccine of the present invention can be prepared in the form of sterilized solution or suspension for injection, or in the form of freeze-dried formula using freeze-drying technique. The freeze-dried vaccine of the present invention is supposed to be maintained typically at about 4°C and can be restored in a stabilizing solution containing or not containing an adjuvant such as saline or/and HEPES.

To accomplish the present invention, the effective dose of the vaccine for administration is determined by considering administration method, administration frequency, specific disease under treatment, severity of disease, disease history, whether or not a patient is under co-treatment with other drugs, age, height, weight, health condition and other physical conditions of a patient, but not always limited thereto. In general, as the weight of a patient under treatment increases, the dose of this preparation is preferably increased.

The vaccine can be administered by effective dose to induce immune response in a patient. For example, the vaccine can be administered to human once or a few times a day by the dosage of $1 \times 10^3 \sim 1 \times 10^9$ cells/kg, and more preferably $1 \times 10^4$ cells/kg $\sim 1 \times 10^8$ cells/kg. To prepare a peripheral blood mononuclear cell vaccine loaded with alpha-galactosylceramide, a medium has to be supplemented with alpha-galactosylceramide at the concentration of $1 \sim 2 \ \mu g/m\ell$ per $1 \times 10^6 \sim 1 \times 10^7$ peripheral blood mononuclear cells/ml. To prepare a peripheral blood mononuclear cell vaccine loaded with alpha-galactosylceramide and peptides, a medium has to be supplemented with alpha-galactosylceramide at the concentration of $1 \sim 2 \ \mu g/m\ell$ per $1 \times 10^6 \sim 1 \times 10^7$ peripheral blood mononuclear cells/ml and peptide at the concentration of $1 \sim 10 \ \mu g/m\ell$ per $1 \times 10^6 \sim 1 \times 10^7$ peripheral blood mononuclear cells/ml.

Alpha-galactosylceramide does not appear to induce toxicity in rodents and monkeys (Nakagawa et al., Cancer Res 58:1202-1207, 1998). No adverse effects were reported even in mice injected with 2200 $\mu g$/kg of $\alpha$GalCer (Giaccone et al., Clin Cancer Res 8:3702, 2002). In ongoing clinical trials, some side effects, such as mild headache, have been reported with systemic administration of $\alpha$GalCer (Mie Nieda et al., Blood 103:383-389, Giaccone et al., Clin Cancer Res 8:3702, 2002), but these could be prevented by administration of paracetamol, and mild systemic side effects do not necessarily occur in these subjects (Giaccone et al., Clin Cancer Res 8:3702, 2002).

The present invention also provides a pharmaceutical composition for preventing or treating cancer, which comprises peripheral blood mononuclear cells loaded with natural killer T cell ligands and antigens as an active ingredient.

The present invention also provides a use of peripheral blood mononuclear cells loaded with natural killer T cell ligands and cancer antigens for preventing or treating cancer.

The present invention also provides a method for preventing or treating cancer by administering an effective amount of peripheral blood mononuclear cells loaded with natural killer T cell ligands and antigens to a subject.

In addition, the present invention provides a method for producing an anticancer immunotherapeutic or preventive vaccine comprising peripheral blood mononuclear cells loaded with natural killer T cell ligands and antigens.

The method comprises the following steps:

(a) a step of obtaining peripheral blood mononuclear cells;
(b) a step of removing red blood cells; and
(c) a step of delivering natural killer T cell ligands and antigens to peripheral blood mononuclear cells.

In step (a), the peripheral blood mononuclear cells can be obtained, for example, using a known leukapheresis technique. Herein, peripheral blood mononuclear cells may include T cells, B cells, natural killer cells, monocytes, and some red blood cells, but may not include neutrophils, eosinophils, or basophils.

In step (b), the red blood cells can be removed by any method known in the art, for example, using a suitable lysis buffer.

In step (c), the natural killer T cell ligands may be added to a culture medium (e.g., a culture medium for peripheral blood mononuclear cells) and delivered to the peripheral blood mononuclear cells through culture. In step (c), the antigens can be delivered by a recombinant virus. The virus introduced into peripheral blood mononuclear cells for antigen expression includes adenovirus, retrovirus, vaccinia virus, Pox virus, and Sindbis virus, but not always limited thereto.

In addition to the method using viruses, the following methods can be applied for antigen gene delivery: 1) a method of transducing DNA by binding it to liposomes to protect the DNA from enzymatic degradation or to allow it to be taken up into endosomes, 2) a method of increasing the efficiency of DNA delivery into cells by combining a molecular conjugate consisting of a protein or a synthetic ligand to the DNA (ex: asialoglycoprotein, transferrin, polymeric IgA), 3) a method for delivering antigen genes by increasing the efficiency of DNA delivery into cells through a novel DNA delivery system using PTD (protein transduction domain) (ex: Mph-1), and 4) In addition to the above methods, peptides can be used or antigenic proteins can be applied to peripheral blood mononuclear cells to cause the peripheral blood mononuclear cells to present the antigen.

The preparation method above eliminates the need for a process of removing other immune cells to obtain each immune cell, unlike the existing method of using each immune cell, which reduces the time and cost of vaccine manufacturing and increases the manufacturing volume.

The present inventors produced a vaccine with significantly improved immune function by changing the immunogenicity of peripheral blood mononuclear cells through $\alpha$-GC loading and antigen delivery to peripheral blood mononuclear cells, thereby allowing peripheral blood mononuclear cells to present antigens.

Antigen delivery using viral vectors is suitable for large-scale production of cell therapy agents targeting cancers expressing specific cancer antigens by delivering cancer antigens to cells with high efficiency. When a virus capable of expressing the above antigen is used, the entire antigen can be introduced, so it is applicable to all people without being limited to a specific haplotype of the major histocompatibility complex, and has the advantage of being able to induce not only a cellular immune response but also a humoral immune response. On the other hand, viral vectors are limited in their application to personalized cancer therapy.

The method of antigen delivery through peptide loading, when used clinically, is limited to the haplotype of an individual's major histocompatibility complex (MHC), so it cannot be used universally, and has the disadvantage of presenting only a single epitope. However, neoepitopes, the peptide sequences that are created by highly tumor-specific mutations that exist only in cancer cells, not normal cells, and can induce cancer cell-specific immune responses, are known as ideal targets for personalized anticancer immunotherapy for cancer patients. Anticancer immunotherapy using an immune cell vaccine that delivers neoepitopes discovered for personalized treatment for cancer patients is expected to induce the generation of more cancer cell-specific T cells than conventional immunotherapy. It can also induce a strong anticancer treatment effect while minimizing the side effects of anticancer treatment by preventing damage to normal cells.

In the present invention, peripheral blood mononuclear cells were isolated from a mouse, loaded with $\alpha$-GC, and an antigen was delivered in two ways (antigen gene transfer to a viral vector expressing the antigen or peptide loading) to produce a peripheral blood mononuclear cells vaccine.

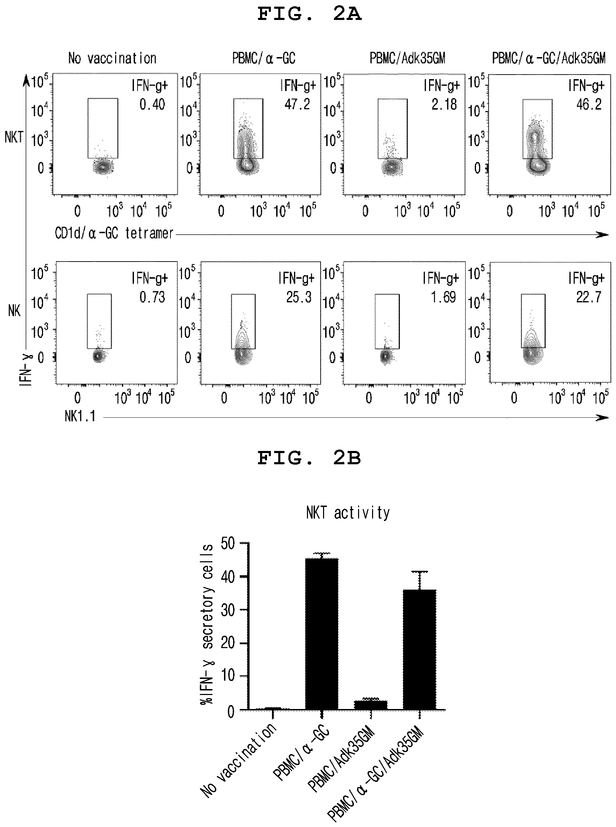

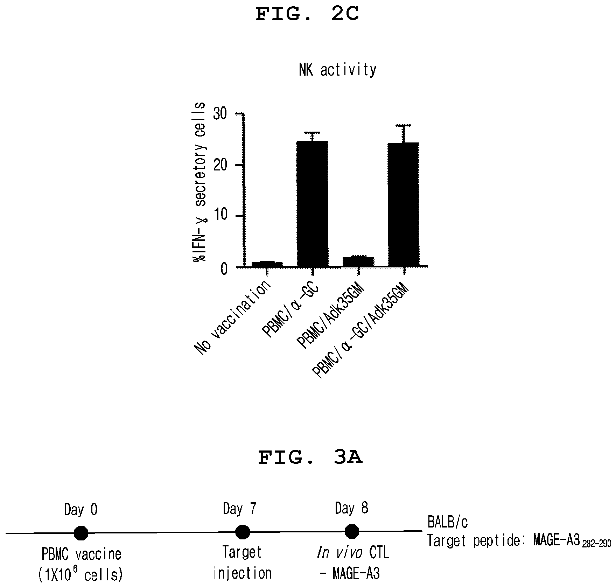

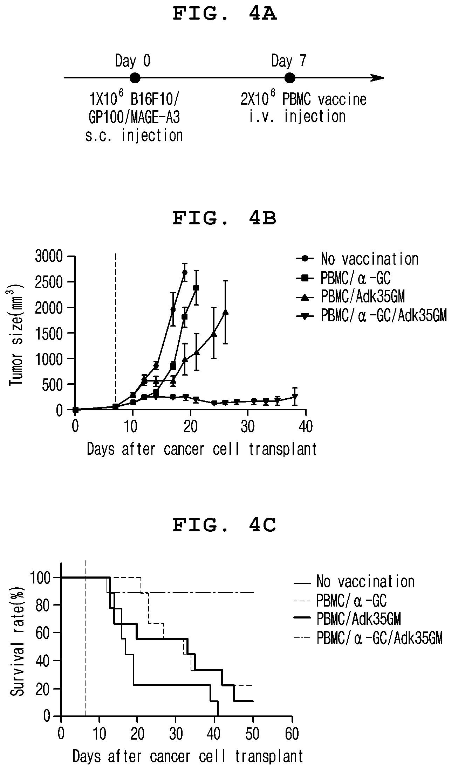

First, a peripheral blood mononuclear cell vaccine in which $\alpha$-GC was loaded and the antigen gene was delivered by an adenovirus vector expressing the antigen was produced, and whether administration of the vaccine could activate natural killer T cells and natural killer cells in the body was confirmed. As a result, it was confirmed that natural killer T cells and natural killer cells were activated in the bodies of mice administered with the peripheral blood mononuclear cells loaded with $\alpha$-GC and the peripheral blood mononuclear cells loaded with $\alpha$-GC and delivered with cancer antigens GP100 and MAGE-A3 via adenovirus (see Figures 2A to 2C). In addition, an *in vivo* CTL assay was performed to determine whether the vaccine could induce a cytotoxic immune response by activating antigen-specific cytotoxic T lymphocytes. As a result, it was confirmed that an effective cytotoxic response was induced by the peripheral blood mononuclear cell vaccine loaded with $\alpha$-GC and adenovirus-delivered cancer antigens (GP100 and MAGE-A3) (see Figures 3A to 3C). In addition, the efficacy of the vaccine in treating solid cancer was confirmed. As a result, it was confirmed that the vaccine exhibited significant anticancer effect, suppressing tumor size and increasing mouse survival rate (see Figures 4A to 4C).

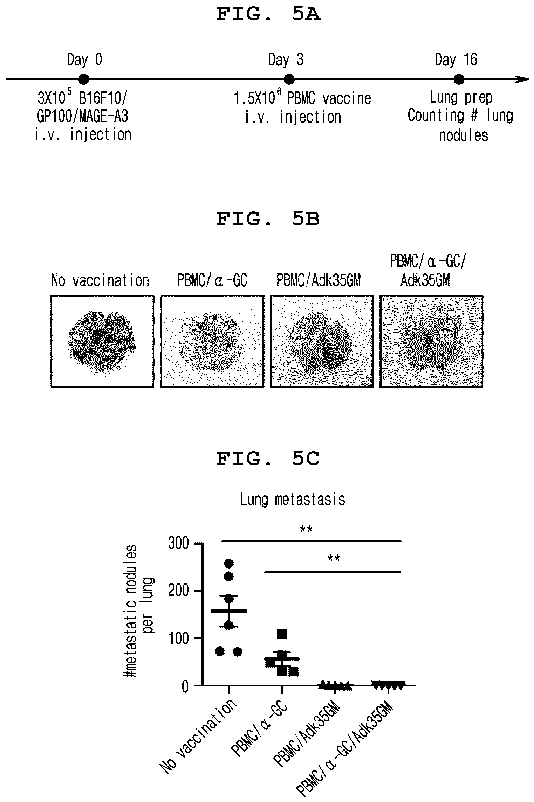

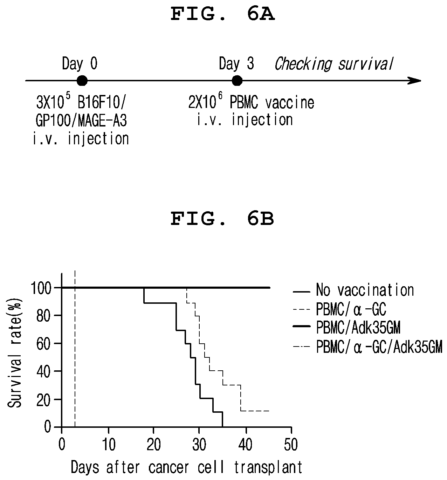

In addition, the therapeutic effect of the above vaccine on lung metastatic cancer was investigated. As a result, in both cell groups where cancer antigens were introduced via adenovirus, almost no cancer tissue was observed (see Figures 5A to 5C), and the survival rate of mice was significantly improved (see Figures 6A and 6B).

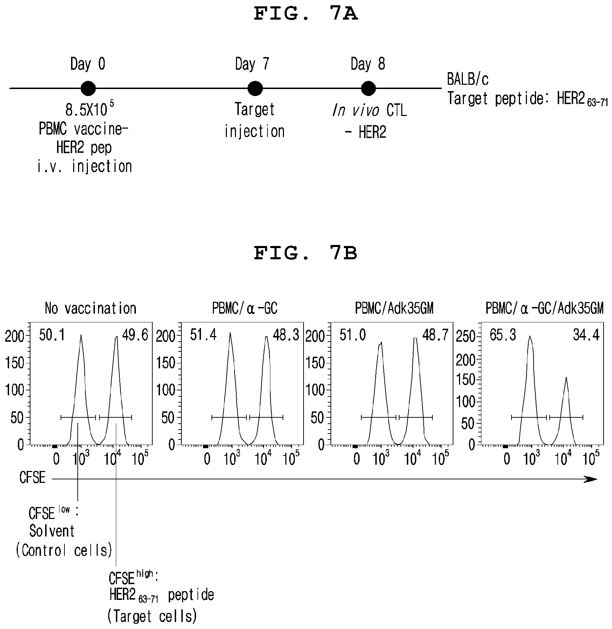

Next, the present inventors produced a peripheral blood mononuclear cell vaccine loaded with $\alpha$-GC and antigen peptides, and confirmed whether cytotoxic T cell responses were induced by administration of the vaccine. As a result, it was confirmed that an effective cytotoxic response was induced by the peripheral blood mononuclear cell vaccine loaded with $\alpha$-GC and HER2$_{63-71}$ peptide (see Figures 7A to 7C).

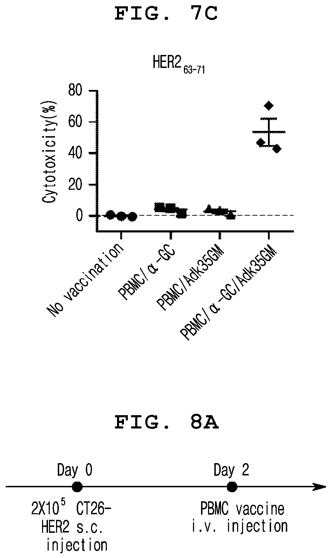

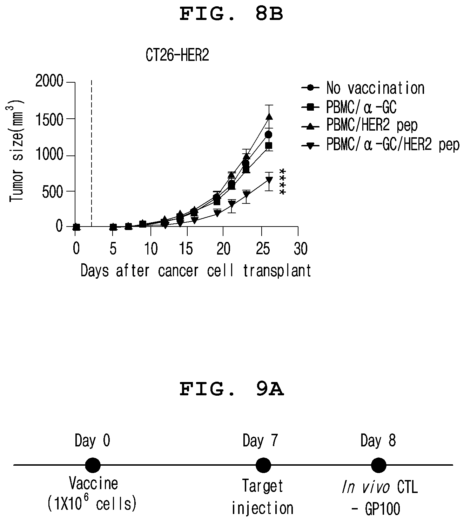

In addition, the anticancer effect of the peripheral blood mononuclear cell vaccine loaded with $\alpha$-GC and cancer antigen peptides was investigated, and as a result, it was confirmed that the tumor size was suppressed (see Figures 8A and 8B).

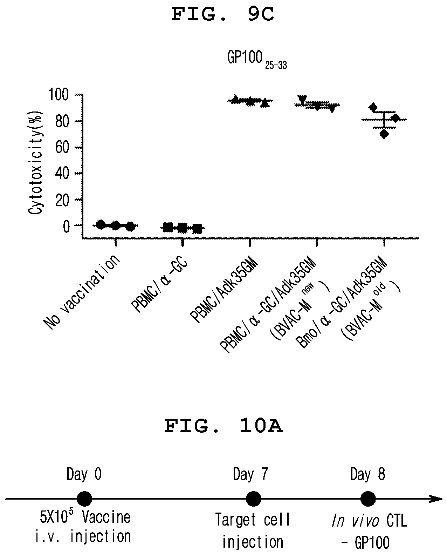

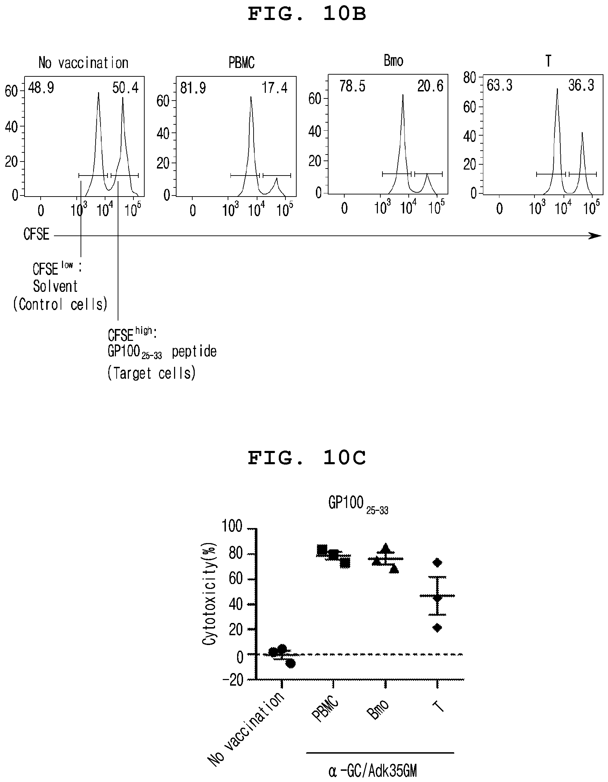

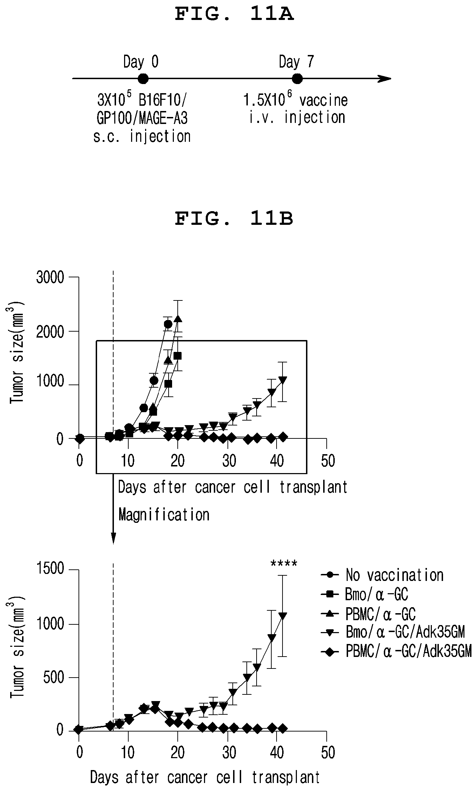

The anticancer effects of a vaccine composed of the peripheral blood mononuclear cells of the present invention and a vaccine composed of other immune cells were compared. First, the present inventors compared the ability of the peripheral blood mononuclear cell vaccine and the B cell/monocyte vaccine to induce cytotoxic T cell immune responses. As a result, Cancer antigen-specific cytotoxicity was observed in both groups administered with the above two types of vaccines, and slightly higher cytotoxicity was confirmed in the group administered with the peripheral blood mononuclear cell vaccine (Figures 9A to 9C). In addition, the ability of the peripheral blood mononuclear cell vaccine, the B cell/monocyte vaccine, and the T cell vaccine to induce cytotoxic T cell immune responses was compared. As a result, it was confirmed that cytotoxic T cell immune responses specific to cancer antigens were induced not only by peripheral blood mononuclear cells, B cells/monocytes, but also by T cells (see Figures 10A and 10C). In particular, it was confirmed that the peripheral blood mononuclear cell vaccine exhibited a strong anticancer therapeutic effect that continuously suppressed cancer growth compared to the B cell/monocyte vaccine (Figures 11A and 11B). This seems to be because various immune cells present in peripheral blood mononuclear cells interact complementarily to induce an enhanced anticancer therapeutic response.

Hereinafter, the present invention will be described in detail by the following experimental examples.

However, the following experimental examples are only for illustrating the present invention, and the contents of the present invention are not limited thereto.

Example 1: Production of peripheral blood mononuclear cell vaccine

<1-1> Isolation and purification of mouse T cells, B cells/monocytes, and natural killer cells

To isolate each of the constituent cells of peripheral blood mononuclear cells from mice, the mouse spleen was collected and then homogenized. After lysing red blood cells using ACK lysing buffer (Gibco), T cells expressing CD4 or $CD8\alpha$ on the cell surface and B cells/monocytes expressing B220 or CD11b were isolated using microbeads (Miltenyibiotec). After the isolation of T cells and B cells/monocytes, CD49b+ natural killer cells were obtained from the remaining spleen cells using anti-CD49b microbeads (Miltenyibiotec). Since the amount of immune cells that can be obtained from mouse blood is limited, immune cells were isolated from the spleen. However, due to the composition of immune cells in blood and spleen being different, to match the composition of blood, each immune cell was mixed (T cells : B cells/monocytes : natural killer cells = 5:4:1) and proportioned to resemble human peripheral blood mononuclear cells.

<1-2> Construction of peripheral blood mononuclear cell vaccine in which antigens are delivered by viral vectors expressing the antigens

The peripheral blood mononuclear cells separated and purified as described above were placed in a medium containing serum along with $\alpha$-GC (1 $\mu$g/mL), solvent (DMSO), and/or adenovirus for cancer antigen gene delivery (200 MOI). The cells were centrifuged (2,000 rpm, 20°C, 90 minutes) in a cell culture plate, and cultured in a $CO_2$ incubator (37°C, 80~95% relative humidity, and 5% $CO_2$ concentration) to produce a peripheral blood mononuclear cell vaccine. The peripheral blood mononuclear cell vaccine was washed three times with Dulbecco's phosphate buffered saline (DPBS, Welgene), dissolved in DPBS, and administered into the tail vein of mice.

<1-3> Construction of peripheral blood mononuclear cell vaccine loaded with antigen peptides

Viral vectors are suitable gene delivery vehicles for the large-scale production of cell therapeutics targeting cancer cells expressing specific cancer antigens, as they deliver cancer antigens common to cancer cells with high efficiency. However, viral vectors have limitations in applying personalized treatment for cancer patients using neoepitopes, so the present inventors have constructed a peripheral blood mononuclear cell vaccine using antigen delivery via peptide loading.

Specifically, the peripheral blood mononuclear cells isolated from mice by the method of Example <1-1> were treated with $\alpha$-GC (1 $\mu$g/mL) or solvent (DMSO), placed in a medium containing serum, and cultured in a $CO_2$ incubator for 15 hours (37°C, 80~95% relative humidity, and 5% $CO_2$ concentration). Then, a peripheral blood mononuclear cell vaccine was prepared by adding $HER2_{63-71}$ peptide (2 $\mu$g/mL) to the cells and further incubating them in a 37°C $CO_2$ incubator for 2 hours. The prepared peripheral blood mononuclear cell vaccine was washed three times with DPBS, dissolved in DPBS, and administered into the tail vein of mice.

Comparative Example 1: Production of B cell/monocyte vaccine

To isolate mouse B cells/monocytes, the mouse spleen was collected and then homogenized. After lysing red blood cells using ACK lysing buffer (Gibco), B cells/monocytes expressing B220 or CD11b were isolated using microbeads. Antigens were delivered to the isolated B cells/monocytes using the same method as in Example <1-2> or <1-3>.

Comparative Example 2: Production of T cell vaccine

To isolate mouse T cells, the mouse spleen was collected and then homogenized. After lysing red blood cells using ACK lysing buffer (Gibco), T cells expressing CD4 or $CD8\alpha$ on the cell surface were isolated using microbeads. Antigens were delivered to the isolated T cells using the same method as in Example <1-2> or <1-3>.

Example 2: Production of human peripheral blood mononuclear cell vaccine

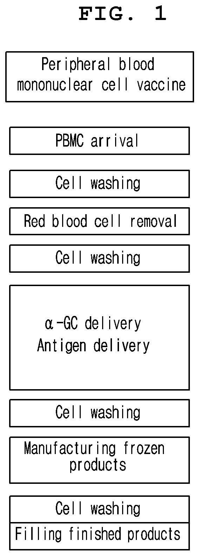

The manufacturing process of the human peripheral blood mononuclear cell vaccine of the present invention is shown in Figure 1.

Specifically, human peripheral blood mononuclear cells obtained by leukapheresis were lysed with ACK lysing buffer (Lonza) to lyse red blood cells, and treated with $\alpha$-GC (1 $\mu$g/mL) and an antigen-expressing viral vector (25 MOI), followed by culturing the cells in a $CO_2$ incubator at 37°C and 5% $CO_2$ concentration for 15 hours to produce a peripheral blood mononuclear cell vaccine. This eliminates the process of removing T cells from the existing manufacturing process, which has the advantage of saving time, manpower and cost, and increasing production volume.

Experimental Example 1: Confirmation of effectiveness of peripheral blood mononuclear cell vaccine in which antigens are delivered by viral vectors expressing antigens

<1-1> Confirmation of induction of activation of natural killer T cells and natural killer cells

The present inventors confirmed whether natural killer T cells and natural killer cells in the body could be activated through the peripheral blood mononuclear cell vaccine administration.

Specifically, the peripheral blood mononuclear cells obtained from C57BL/6 mice were used to make $\alpha$-GC-loaded peripheral blood mononuclear cells (PBMC/$\alpha$-GC), peripheral blood mononuclear cells transduced with adenovirus Adk35GM for delivery of cancer antigens (human GP100 and MAGE-A3) (PBMC/Adk35GM), $\alpha$-GC-loaded and adenovirus Adk35GM-transduced peripheral blood mononuclear cells (PBMC/$\alpha$-GC/Adk35GM), and $1 \times 10^6$ peripheral blood mononuclear cells were administered intravenously. After 6 hours, the level of IFN-$\gamma$ production in natural killer T cells and natural killer cells in the spleen was measured by flow cytometry.

As a result, as shown in Figures 2A to 2C, it was confirmed that natural killer T cells in the body were stimulated and natural killer cells were also activated in the group of mice administered with the peripheral blood mononuclear cells loaded with $\alpha$-GC (PBMC/$\alpha$-GC and PBMC/$\alpha$-GC/Adk35GM), unlike the group of mice administered with the transduced peripheral blood mononuclear cells (PBMC/Adk35GM).

It is thought that the activation of these natural killer T cells and natural killer cells, along with the induction of cytotoxic T cell responses, may contribute to the anticancer effect.

<1-2> Evaluation of ability of peripheral blood mononuclear cell to induce cytotoxic T cell response

To determine whether the adenovirus-transduced peripheral blood mononuclear cell vaccine could induce antigen-specific cytotoxic T cell immune responses, an in vivo CTL assay was performed.

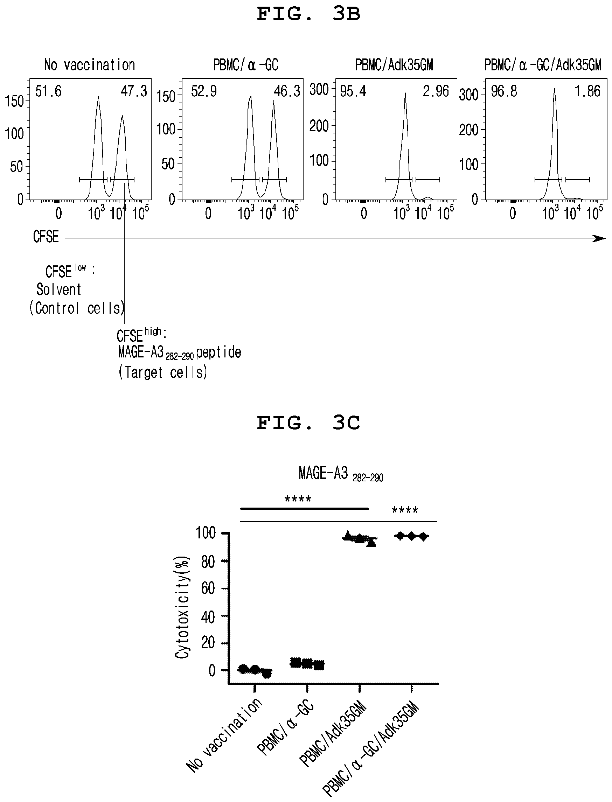

Specifically, the peripheral blood mononuclear cells obtained from BALB/c mice were used to construct $\alpha$-GC-loaded or adenovirus Adk35GM antigen-delivered peripheral blood mononuclear cell vaccines, which were immunized into BALB/c mice and a cytotoxicity assay was performed 7 days later (Figure 3A). First, spleen cells from the same mouse were divided into two groups with equal amounts. The target cells loaded with MAGE-A3$_{282-290}$ peptide were labeled with 3 $\mu$M CFSE (carboxyfluorescein diacetate succinimidyl ester) (CFSE$^{high}$), and the control cells without the peptide were labeled with 0.3 $\mu$M CFSE (CFSE$^{low}$), and then injected into the vaccinated mice in equal amounts. One day later, lysis of the target cells was measured by calculating the CFSE$^{high}$ : CFSE$^{low}$ ratio in mouse spleen cells by flow cytometry (Figure 3B). A lower percentage of the CFSE$^{high}$ cells loaded with antigen peptides indicates a higher antigen-specific cytotoxic T cell immune response.

As a result, as shown in Figure 3C, more than 95% of target cells were killed by MAGE-A3-specific cytotoxic T cell responses only with cancer antigen delivery via Adk35GM adenovirus, and thus a cancer antigen-specific cytotoxic T cell immune response was confirmed.

Based on the results of Experimental Examples <1-1> and <1-2> above, it can be inferred that both innate immune responses, activation of natural killer T cells and natural killer cells, and acquired immune responses, cytotoxic T cell immune responses, can be induced by administration of a peripheral blood mononuclear cell vaccine loaded with $\alpha$-GC and antigen-delivered by an adenoviral vector, and thus exhibit potent anticancer therapeutic effects.

<1-3> Evaluation of anticancer effect of peripheral blood mononuclear cell vaccine

<1-3-1> Therapeutic effect of peripheral blood mononuclear cell vaccine on solid cancer

The present inventors investigated whether the peripheral blood mononuclear cell vaccine administration induces anticancer immunity against solid cancer.

To this end, C57BL/6 mice were immunized with $2 \times 10^6$ PBMC/$\alpha$-GC, PBMC/Adk35GM or PBMC/$\alpha$-GC/Adk35GM 7 days after subcutaneous implantation of $1 \times 10^6$ B16F10/GP100/MAGE-A3 cancer cells expressing GP100 and MAGE-A3 into the flanks of C57BL/6 mice (Figure 4A). Then, tumor size changes and mouse survival rates were measured. The tumor size was calculated using the following equation by measuring the width, length, and height of the tumor using a caliper.

```
(Tumor Size)= (Width) x (Length) x (Height) x (π / 6)
```

As a result, as shown in Figures 4B and 4C, when the peripheral blood mononuclear cell vaccine (PBMC/$\alpha$-GC/Adk35GM) was administered, cancer growth was suppressed and a high survival rate was observed. In contrast, in the mice administered with PBMC/$\alpha$-GC, cancer growth was hardly inhibited and a low survival rate was observed.

Through the above results, it was confirmed that the anticancer therapeutic effect could not be induced by activating natural killer T cells and natural killer cells loaded with $\alpha$-GC alone.

<1-3-2> Therapeutic effect of peripheral blood mononuclear cell vaccine on lung metastatic cancer

The present inventors investigated whether the peripheral blood mononuclear cell vaccine administration induces anticancer immunity against lung metastatic cancer.

To this end, C57BL/6 mice were intravenously injected with $3 \times 10^5$ B16F10/GP100/MAGE-A3 cancer cells, and 3 days later, $1.5 \times 10^6$ PBMC/$\alpha$-GC, PBMC/Adk35GM, or PBMC/$\alpha$-GC/Adk35GM were administered. Sixteen days after intravenous injection of cancer cells, the extent of lung metastasis of the cancer cells was confirmed (Figure 5A).

As a result, as shown in Figures 5B and 5C, in the lungs of mice administered with PBMC/Adk35GM and PBMC/$\alpha$-GC/Adk35GM, two cell populations introduced with cancer antigens via adenovirus, almost no cancer tissue was observed.

Next, the present inventors confirmed the effect of administration of the peripheral blood mononuclear cell vaccine on the survival rate of mice with metastatic cancer.

To this end, C57BL/6 mice were intravenously injected with $3 \times 10^5$ B16F10/GP100/MAGE-A3 cancer cells, and 3 days later, $2 \times 10^6$ PBMC/$\alpha$-GC, PBMC/Adk35GM, or PBMC/$\alpha$-GC/Adk35GM were administered. Then, the survival rate of the mice was tracked (Figure 6A).

As a result, as shown in Figure 6B, similar to the results in Figure 5B, the survival rate of the mouse groups administered with PBMC/Adk35GM and PBMC/$\alpha$-GC/Adk35GM introduced with cancer antigens was significantly improved, and all mice survived for more than 40 days.

Taken together with the results of Experimental Examples <1-2> and <1-3>, it can be seen that delivery of cancer antigens via Adk35GM to peripheral blood mononuclear cells is sufficient to induce a potent cytotoxic T cell response, resulting in a strong anticancer therapeutic effect.

Experimental Example 2: Confirmation of effectiveness of peripheral blood mononuclear cell vaccine loaded with antigen peptides

<2-1> Confirmation of induction of cytotoxic T cell response

The present inventors investigated whether the peripheral blood mononuclear cell vaccine loaded with the antigen peptides of Example <1-2> could induce cytotoxic T cell responses.

C57BL/6 mice were immunized by intravenous administration of peripheral blood mononuclear cells loaded with $\alpha$-GC (PBMC/$\alpha$-GC), peripheral blood mononuclear cells loaded with HER2$_{63-71}$ peptide (PBMC/HER2 pep), peripheral blood mononuclear cells loaded with $\alpha$-GC and HER2$_{63-71}$ peptide together (PBMC/$\alpha$-GC/HER2 pep), and an *in vivo* cytotoxicity assay was performed 7 days later (Figure 7A).

As a result, as shown in Figures 7B and 7C, only in the mouse group administered with PBMC/$\alpha$-GC/HER2 pep, the CFSE$^{high}$ target cells loaded with HER2$_{63-71}$ peptide were mostly lysed. This confirmed that cytotoxic T cells can also be activated by the peripheral blood mononuclear cell vaccine loaded with $\alpha$-GC and peptides.

However, unlike that cytotoxic T cell responses were induced in the group administered with peripheral blood mononuclear cells to which only the antigen was delivered via an adenovirus vector without $\alpha$-GC in Experimental Example <1-2> (Figure 3C), no cytotoxic T cell responses were observed in the group administered with PBMC/HER2 pep loaded with peptide alone (Figure 7C). This suggests that delivering the entire antigen via adenovirus to induce diverse immune responses specific to multiple epitopes is superior to presenting only a single peptide in inducing cytotoxic T cell immune responses.

<2-2> Evaluation of anticancer effect of peripheral blood mononuclear cell vaccine

The present inventors investigated whether the peripheral blood mononuclear cell vaccine loaded with antigen peptides could induce anticancer therapeutic effects.

To this end, BABL/c mice were immunized with $2 \times 10^6$ PBMC/$\alpha$-GC, PBMC/HER2 pep or PBMC/$\alpha$-GC/HER2 pep 3 days after subcutaneous implantation of $2 \times 10^5$ CT26-HER2 cancer cells into the mice (Figure 8A).

As a result, as shown in Figure 8B, when the peripheral blood mononuclear cell vaccine (PBMC/$\alpha$-GC/HER2 pep) was administered, cancer growth was significantly suppressed. In contrast, in the mice administered with PBMC/$\alpha$-GC or PBMC/HER2 pep, tumor growth was hardly inhibited. Therefore, it was revealed that peptide loading alone cannot induce sufficient anticancer therapeutic effects.

When combined with the above results and the anticancer effect data of Example <1-3> (Figures 4 to 6), it was confirmed that the peripheral blood mononuclear cell vaccine prepared by loading $\alpha$-GC and delivering antigen showed a stronger anticancer effect than the cell vaccine loaded with $\alpha$-GC alone or delivered with antigen alone, inhibiting cancer growth and improving survival rate.

Experimental Example 3: Superior anticancer efficacy of peripheral blood mononuclear cell vaccine compared to other immune cell composition vaccines

<3-1> Comparison of immune response induction ability of peripheral blood mononuclear cell vaccine and B cell/monocyte cell vaccine

The ability of the peripheral blood mononuclear cell vaccine to induce cytotoxic T cell immune responses was compared with that of the conventional anticancer cell vaccine composed of B cells/monocytes.

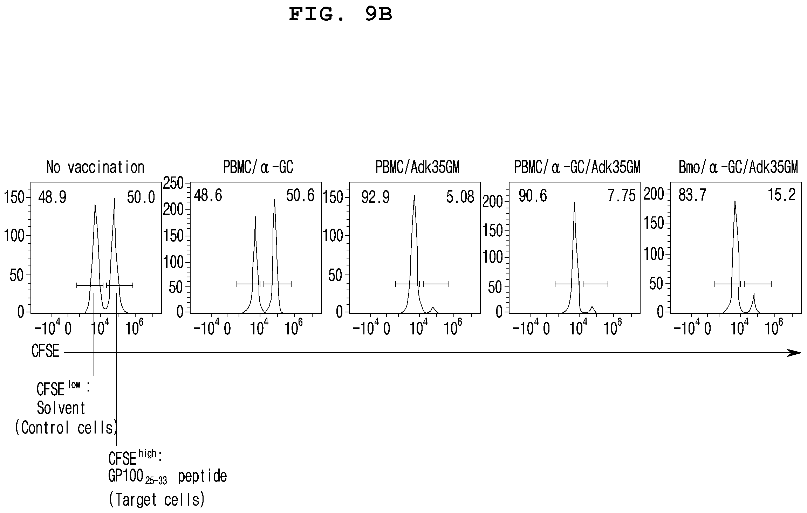

To this end, vaccines were produced by loading $\alpha$-GC or delivering antigen via Adk35GM to B cells/monocytes and peripheral blood mononuclear cells obtained from the spleen of C57BL/6 mice, and immunized into C57BL/6 mice. After 7 days, target cells (CFSE$^{high}$-labeled target cells loaded with GP100$_{25-33}$ peptide: solvent-treated CFSE$^{low}$-labeled control cells = 1:1) were administered to the immunized mice, and an *in vivo* cytotoxicity assay was performed (Figure 9A). One day later, the lysis of GP100$_{25-33}$ peptide-loaded CFSE$^{high}$ target cells was measured by calculating the CFSE$^{high}$ : CFSE$^{low}$ ratio in mouse spleen cells using flow cytometry (Figure 9B).

As a result, as shown in Figure 9C, in both the group administered with the immune cell vaccine composed of B cells/monocytes (Bmo/$\alpha$-GC/Adk35GM) and the group administered with the peripheral blood mononuclear cell vaccine (PBMC/$\alpha$-GC/Adk35GM), more than 80% target cell killing by cytotoxic T cell response specific to the cancer antigen GP100 was observed, and slightly higher cytotoxicity was confirmed in the group administered with the peripheral blood mononuclear cell vaccine.

<3-2> Confirmation of immune response induction ability by cell component of PBMC vaccine

Next, an in vivo cytotoxicity assay was performed to determine whether antigen-specific cytotoxic T cell immune responses could be induced by each type of immune cells comprising the peripheral blood mononuclear cell vaccine.

Specifically, T cells, B cells/monocytes and peripheral blood mononuclear cells obtained from the spleen of C57BL/6 mice were loaded with $\alpha$-GC and delivered with Adk35GM antigen to produce immune cell vaccines. Then, C57BL/6 mice were immunized with the vaccine, and 7 days after immunization, a cytotoxicity test was performed using the same method as in Example <3-1> (Figure 10A).

As a result, as shown in Figures 10B and 10C, the cytotoxicity response was highest in the group administered with the peripheral blood mononuclear cell vaccine (PBMC/$\alpha$-GC/Adk35GM), the group administered with the B cell/monocyte-based immune cell vaccine (Bmo/$\alpha$-GC/Adk35GM), and the group administered with the T cell-based immune cell vaccine (T/$\alpha$-GC/Adk35GM), in that order.

The above results suggest that the tumor antigen GP100-specific cytotoxic T cell immune response is induced not only by B cells/monocytes, which constitute approximately 40% of peripheral blood mononuclear cells, but also by T cells, which constitute half of the cells.

Taken together with the results of Experimental Examples <3-1> and <3-2>, it can be seen that a potent cancer antigen-specific cytotoxic T cell immune response can be induced by T cells, B cells, and monocytes, which account for 90% of the peripheral blood mononuclear cell vaccine loaded with $\alpha$-GC and delivered with the antigen by an adenovirus vector.

<3-3> Comparison of anticancer therapeutic effects of peripheral blood mononuclear cell vaccine and B cell/monocyte cell vaccine

Finally, the anticancer therapeutic effects of the peripheral blood mononuclear cell vaccine and the B cell/monocyte vaccine were compared.

To this end, 3 x 10$^5$ B16F10/GP100/MAGE-A3 cancer cells were subcutaneously transplanted into the flanks of C57BL/6 mice. Seven days later, the mice were immunized with 1.5 x 10$^6$ peripheral blood mononuclear cells (PBMC/$\alpha$-GC, PBMC/$\alpha$-GC/Adk35GM), or B cells/monocytes (Bmo/$\alpha$-GC, Bmo/$\alpha$-GC/Adk35GM), and the changes in tumor size were measured (Figure 11A).

As a result, as shown in Figure 11B, administration of the peripheral blood mononuclear cell vaccine (PBMC/$\alpha$-GC/Adk35GM) and the B cell/monocyte vaccine (Bmo/$\alpha$-GC/Adk35GM) inhibited cancer growth. However, while the solid cancer in the mice administered with Bmo/$\alpha$-GC/Adk35GM subsequently regrew, the cancer in the mice administered with PBMC/$\alpha$-GC/Adk35GM was suppressed for more than 4 weeks, demonstrating a strong anticancer therapeutic effect.

Taken together, the peripheral blood mononuclear cell vaccine and the B cell/monocyte vaccine did not differ in the degree of cytotoxic T cell induction in Experimental Examples <3-1 and <3-2>, but in Experimental Example <3-3>, the peripheral blood mononuclear cell vaccine was confirmed to have a much stronger anticancer therapeutic effect than the B cell/monocyte vaccine. This suggests that different immune cell populations within peripheral blood mononuclear cells interact complementarily to induce enhanced anticancer treatment responses.

**INDUSTRIAL APPLICABILITY**

The vaccine comprising peripheral blood mononuclear cells loaded with natural killer T cell ligands and antigens of the present invention is easy to obtain because there is no need to isolate specific cells from peripheral blood mononuclear cells, and immunization of peripheral blood mononuclear cells loaded with natural killer T cell ligands and antigens not only induces significant levels of natural killer cell and natural killer T cell activation and cytotoxic T lymphocyte responses, but also exhibits synergistic therapeutic effects on malignant tumors, so that it can not only prevent cancer, but can also be applied to cancer treatment through immunotherapy.

**Claims**

1.   An immunotherapeutic and preventive vaccine comprising peripheral blood mononuclear cells loaded with natural killer T cell ligands and antigens.

2.   The immunotherapeutic and preventive vaccine according to claim 1, wherein the peripheral blood mononuclear cells are obtained by removing red blood cells from peripheral blood.

3.   The immunotherapeutic and preventive vaccine according to claim 1, wherein the peripheral blood mononuclear cells are composed of lymphocytes consisting of T cells, B cells and natural killer cells, and monocytes

4.   The immunotherapeutic and preventive vaccine according to claim 1, wherein the natural killer T cell ligand is selected from the group consisting of alpha-galactosylceramide, alpha-glucuronosylceramide, phosphatidylinositol tetramannoside, isoglobotrihexosylceramide, ganglioside GD3, phosphatidylcholine, phosphatidylethanolamine, phosphatidylinositol, sulfitide, beta-galactosylceramide, lipophosphoglycan, glycoinositol phospholipids, beta-anomeric galactosylceramide and alpha-anomeric galactosylceramide, which are analogues of alpha-galactosylceramide, bacterial lipid antigens, and variants of alpha-galactosylceramide.

5.   The immunotherapeutic and preventive vaccine according to claim 1, wherein the antigen is an antigen derived from pathogens including pathogenic bacteria, viruses and parasites, or a cancer antigen.

6.   The immunotherapeutic and preventive vaccine according to claim 5, wherein the antigen derived from pathogenic bacteria is selected from the group consisting of Bordetella pertussis antigens (pertussis toxin, filamentous haemagglutinin, and pertactin), tetanus toxoid, diphtheria toxoid, Helicobacterpylori antigens (capsular polysaccharides of serogroups A, B, C, Y, and W-135), pneumococcal antigen (Streptococcus pnemoniae type 3 capsular polysaccharide), tuberculosis antigen, cholera antigen (cholera toxin B subunit), staphylococcal antigen (staphylococcal enterotoxin B), shigella antigen (shigella polysaccharides), Borrelia sp. antigen, Candida albicans antigen, and Plasmodium antigen.

7.   The immunotherapeutic and preventive vaccine according to claim 5, wherein the antigen derived from viruses is selected from the group consisting of influenza virus antigens (haemagglutinin and neuraminidase), human papilloma virus (HPV) antigen (glycoprotein), vesicular stomatitis virus antigen (vesicular stomatitis virus glycoprotein), cytomegalovirus (CMV) antigen, hepatitis virus antigens (hepatitis A(HAV), B(HBV), C(HCV), D(HDV) and G(HGV) antigens) (core antigen and surface antigen), respiratory synctytial virus (RSV) antigen, herpes simplex virus antigen, human immunodeficiency virus (HIV) antigens (GP-120, GP-160, p18, Tat, Gag, Pol, Env) and combinations thereof.

8.   The immunotherapeutic and preventive vaccine according to claim 5, wherein the cancer antigen is at least one selected from the group consisting of gp100, melanoma antigen gene (MAGE), human papilloma virus (HPV) E6/E7, tyrosinase, tyrosinase-related protein-1 (TRP-1), tyrosinase-related protein-2 (TRP-2), murinoglobulin 1 (MUC-1), carcinoembryonic antigen (CEA), p53, $\alpha$-fetoprotein, breast cancer protein expressed by Her-2/neu, proteinase 3, WT-1, PAP, PSA, PSMA, G250, BAGE, GAGE, NY-ESO- 1, MART-1, MCIR, Ig Idiotype, CDK4, caspase-8, $\beta$-catenin, CIA, BCR/ABL, EBV LMP2a, HCV, HHV-8, 5T4, and tumor-specific mutation-derived neoantigens.

9. The immunotherapeutic and preventive vaccine according to claim 1, wherein the antigen is in the form of a peptide, a lipopolysaccharide, a polysaccharide, a glycoprotein, or a polynucleotide including DNA and RNA.

10. The immunotherapeutic and preventive vaccine according to claim 8, wherein the cancer is selected from the group consisting of liver cancer, thyroid cancer, testicular cancer, bone cancer, glioblastoma, oral cancer, ovarian cancer, brain tumor, multiple myeloma, gallbladder cancer, biliary tract cancer, colon cancer, head and neck cancer, lymphoma, bladder cancer, leukemia, esophageal cancer, kidney cancer, stomach cancer, breast cancer, cervical cancer, prostate cancer, rectal cancer, spinal cord tumor, pancreatic cancer, salivary gland cancer, lung cancer, skin cancer, laryngeal cancer, melanoma, acute myeloid leukemia, neuroblastoma, retinoblastoma, and colorectal cancer.

11. The immunotherapeutic and preventive vaccine according to claim 1, wherein the antigen is introduced and expressed by a recombinant virus.

12. The immunotherapeutic and preventive vaccine according to claim 11, wherein the recombinant virus is adenovirus, retrovirus, vaccinia virus, Pox virus, or Sindbis virus with introduced genes expressing antigens.

13. A method for producing an anticancer immunotherapeutic and preventive vaccine comprising the following steps:

    (a) a step of obtaining peripheral blood mononuclear cells;
    (b) a step of removing red blood cells; and
    (c) a step of delivering natural killer T cell ligands and antigens to peripheral blood mononuclear cells.

14. An anticancer immunotherapeutic and preventive vaccine prepared by the method of claim 13.

15. A pharmaceutical composition for preventing and treating cancer, comprising peripheral blood mononuclear cells loaded with natural killer T cell ligands and cancer antigens as an active ingredient.

16. A use of peripheral blood mononuclear cells loaded with natural killer T cell ligands and cancer antigens for preventing or treating cancer.

17. A method of preventing or treating cancer, comprising administering an effective amount of peripheral blood mononuclear cells loaded with natural killer T cell ligands and cancer antigens to a subject.

FIG. 1

```
┌─────────────────────────────┐
│    Peripheral blood         │
│ mononuclear cell vaccine    │
└─────────────────────────────┘

┌─────────────────────────────┐
│        PBMC arrival         │
└─────────────────────────────┘

┌─────────────────────────────┐
│        Cell washing         │
└─────────────────────────────┘

┌─────────────────────────────┐
│    Red blood cell removal   │
└─────────────────────────────┘

┌─────────────────────────────┐
│        Cell washing         │
└─────────────────────────────┘

┌─────────────────────────────┐
│                             │
│        α-GC delivery        │
│       Antigen delivery      │
│                             │
└─────────────────────────────┘

┌─────────────────────────────┐
│        Cell washing         │
└─────────────────────────────┘

┌─────────────────────────────┐
│    Manufacturing frozen     │
│          products           │
└─────────────────────────────┘

┌─────────────────────────────┐
│        Cell washing         │
├─────────────────────────────┤
│  Filling finished products  │
└─────────────────────────────┘
```

## FIG. 2A

## FIG. 2B

FIG. 2C

**NK activity**

FIG. 3A

## FIG. 3B

No vaccination | PBMC/α-GC | PBMC/Adk35GM | PBMC/α-GC/Adk35GM

CFSE

CFSE$^{low}$:
Solvent
(Control cells)

CFSE$^{high}$:
MAGE-A3$_{282-290}$ peptide
(Target cells)

## FIG. 3C

MAGE-A3$_{282-290}$

Cytotoxicity(%)

No vaccination | PBMC/α-GC | PBMC/Adk35GM | PBMC/α-GC/Adk35GM

## FIG. 4A

Day 0

Day 7

1X10$^6$ B16F10/
GP100/MAGE-A3
s.c. injection

2X10$^6$ PBMC vaccine
i.v. injection

## FIG. 4B

- No vaccination
- PBMC/α-GC
- PBMC/Adk35GM
- PBMC/α-GC/Adk35GM

## FIG. 4C

— No vaccination
--- PBMC/α-GC
— PBMC/Adk35GM
--- PBMC/α-GC/Adk35GM

## FIG. 5A

| Day 0 | Day 3 | Day 16 |
|-------|-------|--------|
| $3\times10^5$ B16F10/ GP100/MAGE-A3 i.v. injection | $1.5\times10^6$ PBMC vaccine i.v. injection | Lung prep Counting # lung nodules |

## FIG. 5B

No vaccination   PBMC/α-GC   PBMC/Adk35GM   PBMC/α-GC/Adk35GM

## FIG. 5C

Lung metastasis

## FIG. 6A

Day 0            Day 3    *Checking survival*

$3 \times 10^5$ B16F10/
GP100/MAGE-A3
i.v. injection

$2 \times 10^6$ PBMC vaccine
i.v. injection

## FIG. 6B

— No vaccination
--- PBMC/α-GC
— PBMC/Adk35GM
--- PBMC/α-GC/Adk35GM

Survival rate(%)

Days after cancer cell transplant

FIG. 7A

Day 0        Day 7       Day 8

BALB/c
Target peptide: HER2$_{63-71}$

$8.5 \times 10^5$
PBMC vaccine-
HER2 pep
i.v. injection

Target
injection

*In vivo* CTL
- HER2

FIG. 7B

No vaccination     PBMC/α-GC     PBMC/Adk35GM     PBMC/α-GC/Adk35GM

CFSE

CFSE$^{low}$:
Solvent
(Control cells)

CFSE$^{high}$:
HER2$_{63-71}$ peptide
(Target cells)

FIG. 7C

HER2$_{63-71}$

FIG. 8A

FIG. 8B

CT26-HER2

FIG. 9A

Day 0
Vaccine
(1X10$^6$ cells)

Day 7
Target
injection

Day 8
*In vivo* CTL
- GP100

FIG. 9B

| No vaccination | PBMC/α-GC | PBMC/Adk35GM | PBMC/α-GC/Adk35GM | Bmo/α-GC/Adk35GM |
|---|---|---|---|---|
| 48.9 / 50.0 | 48.6 / 50.6 | 92.9 / 5.08 | 90.6 / 7.75 | 83.7 / 15.2 |

CFSE

CFSE$^{low}$ :
Solvent
(Control cells)

CFSE$^{high}$:
GP100$_{25-33}$ peptide
(Target cells)

EP 4 552 646 A1

FIG. 9C

GP100$_{25-33}$

FIG. 10A

| Day 0 | Day 7 | Day 8 |
|---|---|---|
| 5X10$^5$ Vaccine i.v. injection | Target cell injection | *In vivo* CTL – GP100 |

FIG. 10B

No vaccination | PBMC | Bmo | T

48.9  50.4 | 81.9  17.4 | 78.5  20.6 | 63.3  36.3

CFSE →

CFSE$^{low}$ :
Solvent
(Control cells)

CFSE$^{high}$:
GP100$_{25-33}$ peptide
(Target cells)

FIG. 10C

GP100$_{25-33}$

Cytotoxicity(%)

No vaccination  PBMC  Bmo  T

α-GC/Adk35GM

## FIG. 11A

Day 0 — 3X10$^5$ B16F10/GP100/MAGE-A3 s.c. injection

Day 7 — 1.5X10$^6$ vaccine i.v. injection

## FIG. 11B

Tumor size(mm$^3$) vs Days after cancer cell transplant

Magnification

****

- No vaccination
- Bmo/α-GC
- PBMC/α-GC
- Bmo/α-GC/Adk35GM
- PBMC/α-GC/Adk35GM

## INTERNATIONAL SEARCH REPORT

| International application No. |
| --- |
| **PCT/KR2023/013932** |

| A. | CLASSIFICATION OF SUBJECT MATTER |
| --- | --- |
| | **A61K 39/00**(2006.01)i; **A61K 35/15**(2015.01)i; **A61K 35/17**(2015.01)i |

According to International Patent Classification (IPC) or to both national classification and IPC

| B. | FIELDS SEARCHED |
| --- | --- |

Minimum documentation searched (classification system followed by classification symbols)

A61K 39/00(2006.01); A61K 35/17(2015.01); A61K 39/12(2006.01); A61K 39/395(2006.01); A61P 31/12(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 말초혈액단핵세포(peripheral blood mononuclear cell), NKT(natural killer T cell), 항원(antigen), 리간드(ligand), 백신(vaccine), 알파-갈락토실세라마이드(alpha-galactosylceramide)

| C. | DOCUMENTS CONSIDERED TO BE RELEVANT | |
| --- | --- | --- |

| Category* | Citation of document, with indication, where appropriate, of the relevant passages | Relevant to claim No. |
| --- | --- | --- |
| Y | KR 10-2009-0051598 A (SEOUL NATIONAL UNIVERSITY INDUSTRY FOUNDATION) 22 May 2009 (2009-05-22)<br>See abstract; paragraphs [0043]-[0045]; example 1; and claims 1-11. | 1-16 |
| Y | KUMAR, S. et al. Ex vivo antigen-pulsed PBMCs generate potent and long lasting immunity to infection when administered as a vaccine. Vaccine. 2017, vol. 35, pp. 1080-1086.<br>See abstract; and pages 1080-1081, 1084 and 1086. | 1-16 |
| Y | MOTOHASHI, S. et al. A phase I-II study of α-galactosylceramide-pulsed IL-2/GM-CSF-cultured peripheral blood mononuclear cells in patients with advanced and recurrent non-small cell lung cancer. The Journal of Immunology. 2009, vol. 182, no. 4, pp. 2492-2501.<br>See abstract; and pages 2493 and 2499-2500. | 1-16 |
| DY | KR 10-0809873 B1 (SEOUL NATIONAL UNIVERSITY INDUSTRY FOUNDATION) 06 March 2008 (2008-03-06)<br>See abstract; and claims 1 and 3-9. | 1-16 |

☑ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **15 December 2023** | **18 December 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2023/013932**

**C.  DOCUMENTS CONSIDERED TO BE RELEVANT**

| Category* | Citation of document, with indication, where appropriate, of the relevant passages | Relevant to claim No. |
|---|---|---|
| A | KR 10-2122802 B1 (KNU-INDUSTRY COOPERATION FOUNDATION) 16 June 2020 (2020-06-16)<br>See entire document. | 1-16 |

Form PCT/ISA/210 (second sheet) (July 2022)

**INTERNATIONAL SEARCH REPORT**

International application No.

**PCT/KR2023/013932**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **17**
because they relate to subject matter not required to be searched by this Authority, namely:

   Claim 17 pertains to a method for treatment of the human body, and thus pertains to a subject matter on which the International Searching Authority is required to carry out an international search under the provisions of PCT Article 17(2)(a)(i) and PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

INTERNATIONAL SEARCH REPORT
Information on patent family members

| International application No.<br>**PCT/KR2023/013932** |
|---|

| Patent document<br>cited in search report | Publication date<br>(day/month/year) | Patent family member(s) | | Publication date<br>(day/month/year) |
|---|---|---|---|---|
| KR    10-2009-0051598    A | 22 May 2009 | CN | 101861167 A | 13 October 2010 |
| | | CN | 101861167 B | 28 August 2013 |
| | | EP | 2227253 A1 | 15 September 2010 |
| | | EP | 2227253 B1 | 17 May 2017 |
| | | ES | 2641959 T3 | 14 November 2017 |
| | | JP | 2011-503174 A | 27 January 2011 |
| | | JP | 5153885 B2 | 27 February 2013 |
| | | KR | 10-1055666 B1 | 09 August 2011 |
| | | US | 2009-0285851 A1 | 19 November 2009 |
| | | US | 9518126 B2 | 13 December 2016 |
| | | WO | 2009-066824 A1 | 28 May 2009 |
| KR        10-0809873    B1 | 06 March 2008 | BR | PI0621599 A2 | 13 December 2011 |
| | | BR | PI0621599 B1 | 27 July 2021 |
| | | CN | 101426523 A | 06 May 2009 |
| | | CN | 101426523 B | 27 March 2013 |
| | | EP | 2029165 A1 | 04 March 2009 |
| | | EP | 2029165 B1 | 03 December 2014 |
| | | ES | 2529257 T3 | 18 February 2015 |
| | | JP | 2008-527051 A | 24 July 2008 |
| | | JP | 4713638 B2 | 29 June 2011 |
| | | US | 2010-0028380 A1 | 04 February 2010 |
| | | US | 8003093 B2 | 23 August 2011 |
| | | WO | 2007-126163 A1 | 08 November 2007 |
| KR        10-2122802    B1 | 16 June 2020 | KR | 10-2019-0129424 A | 20 November 2019 |
| | | WO | 2019-216543 A1 | 14 November 2019 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220116552
- KR 100809873
- KR 101055666

- KR 1020070105662
- KR 1020090051598
- KR 1020220072485

**Non-patent literature cited in the description**

- **YEONSEOK CHUNG et al.** *OncoImmunology*, 2012, vol. 1 (2), 141-151
- **VAN DER VLIET HJ et al.** *J Immunol Methods.*, 2001, vol. 247 (1-2), 61-72
- Remington's Pharmaceutical Science. Mack Publishing Company

- **NAKAGAWA et al.** *Cancer Res*, 1998, vol. 58, 1202-1207
- **GIACCONE et al.** *Clin Cancer Res*, 2002, vol. 8, 3702
- **MIE NIEDA et al.** *Blood*, vol. 103, 383-389